# EUROPÄISCHE PATENTSCHRIFT

(11) **EP 0 916 680 B1**
(45) Veröffentlichungstag und Bekanntmachung des Hinweises auf die Patenterteilung: **28.01.2004**
(21) Anmeldenummer: 98119105.9
(22) Anmeldetag: 09.10.1998
(51) Int. Cl.: C07K 7/08, C12P 21/02, A61K 38/10

(54) **Lantibiotikum verwandt mit Actagardine, Verfahren zur Herstellung und Verwendung desselben**
Lantibiotic related to Actagardine, process of preparation and use thereof
Lantibiotique apparenté à l'actagardine, son procédé de préparation et utilisation

(30) Priorität: 15.10.1997 DE 19745583
(43) Veröffentlichungstag der Anmeldung: 19.05.1999
(73) Patentinhaber: Aventis Pharma Deutschland GmbH, 65929 Frankfurt am Main (DE)
(72) Erfinder: Vértesy, Laszlo, Dr., 65817 Eppstein (DE); Kogler, Herbert, Dr., 61479 Glashütten (DE); Schiell, Matthias, 65611 Brechen (DE); Wink, Joachim, Dr., 63322 Rödermark (DE)

(56) Entgegenhaltungen:
- EP-A- 0 195 359
- WO-A-94/28726
- US-A- 4 022 884
- ZIMMERMANN N ET AL: "THE TETRACYCLIC LANTIBIOTIC ACTAGARDINE H-NMR AND C-NMR ASSIGNMENTS AND REVISED PRIMARY STRUCTURE" EUROPEAN JOURNAL OF BIOCHEMISTRY, BERLIN, DE, Bd. 228, Nr. 3, 15. März 1995 (1995-03-15), Seiten 786-797, XP000645986 ISSN: 0014-2956
- MALABARBA A ET AL: "SYNTHESIS AND BIOLOGICAL ACTIVITY OF SOME AMIDE DERIVATIVES OF THE LANTIBIOTIC ACTAGARDINE" JOURNAL OF ANTIBIOTICS, JAPAN ANTIBIOTICS RESEARCH ASSOCIATION. TOKYO, JP, Bd. 43, Nr. 9, 1. September 1990 (1990-09-01), Seiten 1089-1097, XP000647318 ISSN: 0021-8820

## Beschreibung

Neues Lantibiotikum verwandt mit Actagardine, Verfahren zur Herstellung und Verwendung derselben.

Die vorliegende Erfindung betrifft ein neues Lantibiotikum verwandt mit Actagardine, besonders ein Lantibiotikum mit der Bezeichnung Ala⁰-Actagardine, ein Verfahren zu dessen Herstellung, von dem Lantibiotikum abgeleitete chemische Derivate und die Verwendung der Lantibiotika als Arzneimittel.

Es ist bereits eine größere Anzahl von Lantibiotika beschrieben worden. Lantibiotika sind polycyclische Peptidantibiotika, die als charakteristisches Merkmal die Aminosäure Lanthionin oder Methyllanthionin enthalten. Es sind mikrobiell gewonnene Naturstoffe, die als antibakterielle Wirkstoffe in der Humantherapie, als Konservierungsstoffe oder als Enzyminhibitoren Verwendung finden ( G. Jung, Angew. Chem. Int. Ed. Engl., 1991, 30, 1051-1068. ).

Zur Behandlung von bakteriellen Infektionskrankheiten wird eine große Zahl von Antibiotika therapeutisch eingesetzt. Die Krankheitserreger werden aber zunehmend resistent gegen die verwendeten Arzneimittel, es droht sogar eine große Gefahr durch sogenannte multiresistente Keime, die nicht nur gegen einzelne Antibiotikagruppen, wie z. B. β-Lactam-Antibiotika oder Glycopeptide oder Macrolide wiederstandsfähig geworden sind, sonderen gleichzeitig mehrere Resistenzen tragen. Es gibt sogar Krankheitserreger, die gegen alle im Handel erhältlichen Antibiotika resistent geworden sind. Infektionskrankheiten, die durch solche Keime verursacht werden, sind nicht mehr therapierbar. Deshalb gibt es einen großen Bedarf an neuen Mitteln, die gegen resistente Keime eingesetzt werden können. Es sind zwar in der Literatur viele Tausend Antibiotika beschrieben worden, die meisten sind jedoch zu toxisch, um als Arzneimittel eingesetzt werden zu können.

Das Actagardine ist ein Lantibiotikum, welches S. Somma et al. in Antimicrob. Agents Chemother. 11, 396-401 erstmals 1977 beschrieben haben. Seine Struktur wurde erst vor kurzem korrekt aufgeklärt ( N. Zimmermann et al., Eur. J. Biochem. 1995, 228, 786- 797 ).

Es ist überraschend gefunden worden, daß die Stämme *Actinoplanes liguriae* und *Actinoplanes garbadiensis* jeweils mindenstens ein neues Antibiotikum zu bilden vermögen, z.B. das Ala⁰-Actagardine, welches nicht nur antibakteriell sehr wirksam, sondern auch gut verträglich ist. Ein Isolat von *Actinoplanes liguriae* wurde bei der Deutschen Sammlung von Mikroorganismen und Zellkulturen GmbH, Mascheroder Weg 1B, 38124 Braunschweig, Deutschland ( hiernach >DSM=), nach den Regeln des Budapester Vertrages am 24.09.97 unter der folgenden Nummer hinterlegt: DSM 11797. Ein Isolat von *Actinoplanes garbadiensis* wurde bei der DSM nach den Regeln des Budapester Vertrages am 24.09.97 unter der folgenden Nummer hinterlegt: DSM 11796.

Entsprechend stellt die Erfindung Verbindungen der Formel I dar, wobei R der Rest einer Aminosäure bedeutet, sowie deren physiologisch verträgliche Salze. R kann der Rest einer substituierten oder unsubstituierten Aminosäure sein in der die Aminogruppe α- bis ω-ständig und in D- oder L-Konfiguration ist. Besonders bevorzugt sind substituierte oder unsubstituierte α-Aminosäuren in D- oder L-Konfiguration.

Vorzugsweise bedeutet R der Rest eine natürliche Aminosäure ausgewält aus: Ala, Gly, Glu, Phe, Pro, Thr. Cys, Met, Trp, Tyr, Asn, Gln, Asp, His, Ile, Leu, Lys, Arg, Ser und Val. Besonders bevorzugt bedeutet die Aminosäure Ala, Ile, Lys, Phe, Val, Glu, Asp, His, Leu, Arg oder Ser und ganz besonders bevorzugt bedeutet die Aminosäure Ala⁰.

R kann auch ein substituierter oder unsubstituierter Diaminoalkansäure-Rest bedeuten wie z. B. 2,4-Diaminobuttersäure (Dab).

Außerdem betrifft die Erfindung eine Verbindung der Summenformel: C₈₄H₁₂₉N₂₁O₂₅S₄ (Ala⁰-Actagardine) erhältlich durch Fermentierung von *Actinoplanes liguriae*, DSM 11797 oder *Actinoplanes garbadiensis*, DSM 11796 bzw. einer ihrer Varianten und/oder Mutanten, in einem Kulturmedium bis sich die Verbindung Ala⁰-Actagardine in der Kulturbrühe anhäuft und durch anschließender Isolierung der Verbindung, sowie deren pharmakologisch verträglichen Salzen.

Die Erfindung betrifft weiterhin chemische Derivate, abgeleitet aus einer Verbindung der Summenformel C₈₄H₁₂₉N₂₁O₂₅S₄ (Ala⁰-Actagardine), erhältlich durch Fermentierung von *Actinoplanes liguriae*, DSM 11797 oder *Actinoplanes garbadiensis* DSM 11796 bzw. einer ihrer Varianten und/oder Mutanten in einem Kulturmedium, bis sich die Verbindung Ala⁰-Actagardine in der Kulturbrühe anhäuft und durch anschließende Isolierung der Verbindung und Überführung in chemische Derivate, sowie deren pharmakologisch verträgliche Salze.

Bevorzugte chemische Derivate sind: Ile⁰-, Lys⁰, Phe⁰-, Val⁰-, Glu⁰ -, Asp⁰ -, His⁰ -, Leu⁰-, Arg⁰ - und Ser⁰-Actagardine. Die Überführung von Ala⁰-Actagardine in die genannten chemischen Derivate können nach dem Fachmann bekannten Methoden hergestellt werden.

Durch die angegebenen Strukturformeln unterscheidet sich das Antibiotikum Ala⁰-Actagardine von literaturbekannten Substanzen. Es sind neben dem Actagardine noch einige Actagardine-Nebenkomponenten beschrieben worden ( US Patent 4,022,884 vom 10. Mai 1976 sowie A. Malabarba et al., J. Antibiotics, 1985, 38, 1506-1511 ), die sich jedoch alle entweder durch die Polarität, auch in Bezug auf das Actagardine oder durch die Aminosäurezusammensetzung oder durch die antimikrobielle Wirksamkeit oder durch weitere physikalischen Eigenschaften von den erfindungsgemäßen Verbindungen unterscheiden.

Der Stamm *Actinoplanes liguriae*, DSM 11797 bildet auf Glukose-, Stärke- oder Glycerinhaltigen Nährlösungen das Actagardine sowie die literaturbekannten Nebenprodukte. Es ist überraschend gefunden worden, daß der selbe Organismus auf schwerer verdaulichen, mannit-enthaltenden Medien in sehr guten Ausbeuten das erfindungsgemäße Antibiotikum NH₂-R-Actagardine, wobei R den Rest einer näturlichen Aminosäure bedeutet, besonders Alanine, produziert, nicht jedoch die beschriebenen Verbindungen, selbst Actagardine nur in Spuren.

Weiterhin betrifft die Erfindung daher ein Verfahren zur Herstellung der Verbindung der Formel I, dadurch gekennzeichnet, daß der Mikroorganismus *Actinoplanes liguriae*, DSM 11797 oder *Actinoplanes garbadiensis*, DSM 11796 bzw. einer ihrer Varianten oder Mutanten in einem wäßrigen Nährmedium kultiviert wird, eine Verbindung der Formel I isoliert und gereinigt wird und gegebenenfalls in ihre pharmakologisch verträglichen Salze überführt wird.

Das besagte Verfahren umfasst die Kultivierung von *Actinoplanes liguriae*, DSM 11797 oder *Actinoplanes garbadiensis*, DSM 11796 seiner Mutanten und/oder Varianten unter aeroben Bedingungen in eine Kohlenstoff- und Stickstoffquelle, anorganischen Salze und Spurenelemente enthaltenden Kulturmedien.

Vorzugsweise wird die Kultivierung bei einer Temperatur zwischen 20 und 35°C und bei einem pH zwischen 4 und 10 durchgeführt.

Außerdem betrifft die Erfindung ein Verfahren zur Herstellung. einer Verbindung der Formel 1, dadurch gekennzeichnet, daß die Verbindung Actagardine mit einer Aminosäure umgesetzt wird.

Beispielsweise kann ein aktivierter Aminosäureester mit der terminalen Aminogruppe des Actagardines umgesetzt werden. Am Aminostickstoff der Aminosäure ist vorzugsweise eine Schutzgruppe, wie z.B. tert.-Butyloxy-carbonyl (Boc-), gebunden um Reaktionen der aktivierten Aminosäureester mit sich selber zu verhindern. Aktivierte Ester sind, z. B., die N-Hydroxysuccinimid-Ester der jeweiligen Aminosäuren. Die Schutzgruppe wird abgespaltet und anschließend wird das Reaktionsgemisch gereinigt.

Der Actinoplanes besitzt oranges Substratmycel und kein Luftmycel. Er bildet die für Actinoplanes charakteristischen Sporangien. Die Zellwand enthält meso-Diaminopimelinsäure sowie Glycin als charakteristische Aminosäuren und Xylose sowie Arabinose als Zucker, dies sind charakteristische Merkmale für die Gattung Actinoplanes.

Anstelle des Stammes DSM 11797 oder 11796 können auch deren Mutanten und Varianten eingesetzt werden, soweit sie die erfindungsgemäßen Verbindungen synthetisieren. Solche Mutanten können in an sich bekannter Weise durch physikalische Mittel, beispielsweise Bestrahlung, wie mit Ultraviolett- oder Röntgenstrahlen, oder chemische Mutagene, wie beispielsweise Ethylmethansulfonat (EMS); 2-Hydroxy-4-methoxy-benzophenon (MOB) oder N-Methyl-N'-nitro-N-nitrosoguanidin (MNNG) erzeugt werden.

Das Screening nach Mutanten und Varianten, die das erfindungsgemäße Antibiotikum produzieren, kann durch Bestimmung der biologischen Aktivität des in der Kulturbrühe angehäuften Wirkstoffes, beispielsweise durch Austesten der antibakteriellen Wirkung erfolgen.

Als bevorzugte Kohlenstoffquelle für die aerobe Fermentation eignen sich assimilierbare, aber schwer verdauliche Kohlenhydrate und Zuckeralkohole, wie Mannit, Inosit sowie kohlenhydrathaltige Naturprodukte, wie z.B.Sojamehl. Als stickstoffhaltige Nährstoffe kommen in Betracht: Aminosäuren, Peptide und Proteine sowie deren Abbauprodukte, wie Peptone oder Tryptone, femer Fleischextrakte, gemahlene Samen, beispielsweise von Mais, Weizen, Bohnen, Hafer, Soja oder der Baumwollpflanze, Destillationsrückstände der Alkoholherstellung, Fleischmehle oder Hefeextrakte, aber auch Ammoniumsalze und Nitrate. An anorganischen Salzen kann die Nährlösung beispielsweise Chloride, Carbonate, Sulfate oder Phosphate der Alkali- oder Erdalkalimetalle, Eisen, Zink, Kobalt und Mangan enthalten.

Die Bildung des Ala(0)-Actagardines verläuft besonders gut z. B. in einer Nährlösung, die etwa 0, 5 bis 5% Mannit vorzugsweise 1 bis 3%, 0,5 bis 5 % Sojamehl, vorzugsweise 1 bis 3 % und eine Spurenelementlösung in einer Konzentration von 0,1 bis 0,5 % vorzugsweise 0,2 bis 0,3 % enthält. Die Spurenelementlösung enthält CaCl₂, Fe III Citrat, MnSO₄, ZnCl₂, CuSO₄, Natriumtetraborat, CoCl₂ und Natriummolybdat.

Die Kultivierung erfolgt aerob, also beispielsweise submers unter Schütteln oder Rühren in Schüttelkolben oder Fermentem, gegebenenfalls unter Einführen von Luft oder Sauerstoff. Die Fermentation kann beispielsweise in Steilbrustflaschen oder Rundkolben verschiedener Volumina, in Glasfermentern oder V₂A-Stahltanks durchgeführt werden. Sie kann in einem Temperaturbereich von etwa 20 bis 35° C, vorzugsweise bei etwa 25 bis 30° C, durchgeführt werden. Der pH-Wert sollte zwischen 4 und 10 liegen, vorteilhaft zwischen 5,5 und 8,5. Man kultiviert den Mikroorganismus unter diesen Bedingungen im allgemeinen über einen Zeitraum von 20 bis 300 Stunden, bevorzugt 24 bis 140 Stunden. Vorteilhaft kultiviert man in mehreren Stufen, d. h. man stellt zunächst eine oder mehrere Vorkulturen in einem flüssigen Nährmedium her, die dann in das eigentliche Produktionsmedium, die Hauptkultur, beispielsweise im Volumenverhältnis 1:10, überimpft werden. Die Vorkultur erhält man z. B. indem man ein Mycel in eine Nährlösung überimpft und etwa 20 bis 120 Stunden, bevorzugt 24 bis 72 Stunden, wachsen läßt. Das Mycel kann beispielsweise erhalten werden, indem man den Stamm etwa 1 bis 40 Tage, bevorzugt 3 bis 10 Tage, auf einem festen oder flüssigen Nährboden, beispielsweise Hefe-Malz-Agar oder Haferflocken-Agar, wachsen läßt.

Der Fermentationsverlauf und die Bildung des erfindungsgemäße Antibiotikums kann entsprechend dem Fachmann bekannten Methoden, wie z. B. durch Austestung der biologischen Aktivität in Bioassays oder durch chromatographische Methoden wie Dünnschichtchromatographie (DC) oder Hochleistungsflüssigkeitschromatographie (HPLC) verfolgt werden.

Das Antibiotikum Ala(0)-Actagardine kann sowohl im Mycel als auch im Kulturfiltrat vorkommen, gewöhnlich befindet sich die Hauptmenge in dem Kulturfiltrat. Es ist deshalb zweckmäßig, die Fermentationslösung durch Filtration oder Zentrifugation zu trennen. Das Filtrat wird mit einem Adsorptionsharz als feste Phase extrahiert. Das Mycel wird zweckmäßigerweise mit Methanol oder Aceton extrahiert, es können aber auch andere Lösungsmittel verwendet werden.

Die Extraktionen können in einem weiten pH-Bereich durchgeführt werden, es ist jedoch zweckmäßig, im neutralen oder schwach sauren Milieu, vorzugsweise zwischen pH 3 und pH 7 zu arbeiten. Die Extrakte können z. B. im Vakuum konzentriert und getrocknet werden.

Eine Methode der Isolierung des erfindungsgemäße Antibiotikums ist die Lösungsverteilung in an sich bekannter Weise.

Eine andere Methode der Reinigung ist die Chromatographie an Adsorptionsharzen wie z. B. an Diaion® HP-20 (Mitsubishi Casei Corp., Tokyo), an Amberlite® XAD 7 (Rohm and Haas, USA), an Amberchrom® CG, (Toso Haas, Philadelphia, USA) oder an ähnlichen. Geeignet sind darüber hinaus zahlreiche Reverse- Phase Träger, z. B. RP₈ und RP₁₈, wie sie z. B. im Rahmen der HochdruckflüssigkeitsChromatographie (HPLC) allgemein bekannt geworden sind.

Eine weitere Reinigungsmöglichkeit für das erfindungsgemäße Antibiotikum besteht in der Verwendung von sog. Normal-Phasen-Chromatographie-Trägem, wie z. B. Kieselgel oder Al₂O₃ oder anderen in an sich bekannter Weise.

Ein alternatives Isolierungsverfahren ist die Verwendung von Molekularsieben, wie z. B. Fractogel® TSK HW-40, Sephadex® G-25 und andere, in an sich bekannter Weise. Es ist darüber hinaus auch möglich, aus angereichertem Material das Ala(0)-Actagardine durch Kristallisation zu gewinnen. Geeignet hierzu sind z. B. organische Lösungsmittel und ihre Gemische, wasserfrei oder mit Wasserzusatz. Ein zusätzliches Verfahren zur Isolierung und Reinigung der erfindungsgemäßen Antibiotika besteht in der Verwendung von Anionenaustauschem, vorzugsweise im pH-Bereich von 4 bis 10 und Kationenaustauschem vorzugsweise im pH-Bereich von 2 bis 5. Besonders geeignet ist hierfür die Verwendung von Pufferlösungen, denen man Anteile von organischen Lösungsmitteln hinzugefügt hat.

Ala(0)-Actagardine, die genannten chemischen Derivate davon sowie die offensichtlichen chemischen Äquivalente derselben können nach dem Fachmann bekannten Methoden in die entsprechenden pharmakologisch verträglichen Salze übergeführt werden.

Offensichtliche chemische Äquivalente der erfindungsgemäßen Verbindungen sind Verbindungen, die einen geringfügigen chemischen Unterschied aufweisen, also die gleiche Wirksamkeit haben oder sich unter milden Bedingungen in die erfindungsgemäßen Verbindungen umwandeln. Zu den genannten Äquivalenten gehören z.B. Ester, Aminoderivate, Komplexe oder Addukte der bzw. mit den erfindungsgemäßen Verbindungen.

Unter pharmakologisch verträglichen Salzen der erfindungsgemäßen Verbindungen versteht man sowohl anorganische als auch organische Salze, wie sie in Remingtons Pharmaceutical Sciences (17. Auflage, Seite 1418 (1985)) beschrieben sind. Als Salze kommen insbesondere Alkali-, Ammonium-, Erdalkalisalze, Salze mit physiologisch verträglichen Aminen und Salze mit anorganischen oder organischen Säuren wie z. B. HCl, HBr, H₂SO₄, Maleinsäure, Fumarsäure in Frage.

Die physikalisch-chemischen sowie spektroskopischen Eigenschaften der erfindungsgemäßen Antibiotika lassen sich wie folgt zusammenfassen:

### Ala(0)-Actagardine:

### Aussehen:

farblose, in Methanol und Wasser lösliche Substanz. Stabil in neutralem und mild alkalischem Milieu, jedoch unbeständig in stark-saurer und stark-alkalischer Lösung.

| | |
|---|---|
| Summenformel | C₈₄H₁₂₉N₂₁O₂₅S₄ |
| Molekulargewicht | 1961,21 |
| ¹H- und ¹³C-NMR | siehe Tabelle 1 und 2 |
| UV-Maxima (log ε) | 280 nm (3,71 ), 288 nm (Schulter) |

**Tabelle 1 :**

| ¹H-NMR spektroskopische Daten von Ala⁰-Actagardine | | | | | |
|---|---|---|---|---|---|
| Amino Säure | HN | Hα | Hβ | Hγ | Andere |
| Ala⁰ | 8.010 | 2.891 | 1.383 | | |
| Ala¹ | 8.564 | 4.727 | 3.397 | - | |
| | | | 2.603 | | |
| Ser² | 8.264 | 4.350 | 3.651 | | OH: 5.102 |
| Gly³ | 8.595 | 3.968 | - | - | |
| | | 3.263 | | | |
| Trp⁴ | 8.147 | 4.475 | 3.311 | | H5: 7.144; H6: |
| | | | | | 7.554; H7: 6.983; |
| | | | | | H8: 7.061; H9: |
| | | | | | 7.333; indol: |
| | | | | | 10.740 |
| | | | 2.978 | | |
| Val⁵ | 7.454 | 4.558 | 2.048 | 0.909 | |
| | | | | 0.856 | |
| Ala⁶ | 8.487 | 4.704 | 2.578 | | |
| | | | 2. 960 | | |
| Abu⁷ | 8.324 | 4.557 | 3.596 | 1.176 | |
| Leu⁸ | 7.636 | 4.626 | 1.418 | 1.482 | δ 0.841 |
| | | | 1.482 | γ-Me: 0.860 | |
| Abu⁹ | 7.604 | 4.747 | 3.570 | 1.207 | |
| Ile¹⁰ | 8.378 | 3.763 | 1.605 | 1.063 | δ-Me: 0.860 |
| | | | β-Me: 0.879 | 1.642 | |
| Glu¹¹ | 8.262 | 3.690 | 2.178 | 2.319 | |
| Ala¹² | 7.325 | 4.553 | 2.559 | - | |
| | | | 2.866 | | |
| Gly¹³ | 8.140 | 3.538 | - | - | |
| | | 4.167 | | | |
| Abu¹⁴ | 7.860 | 4.381 | 3.336 | 1.059 | |
| Val¹⁵ | 7.778 | 4.104 | 2.042 | 0.875 0.873 | |
| Ile¹⁶ | 7.589 | 3.867 | 1.875 | 1.513 | δ-Me: 0.833 |
| | | | β-Me: 0.889 | 1.115 | |
| Ala¹⁷ | 7.577 | 4.488 | 2.594 | | |
| | | | 2.878 | | |
| Ala¹⁸ | 8.181 | 4.036 | 1.232 | | |
| Ala¹⁹ | 8.342 | 4.447 | 2.934 | | |
| | | | 3.069 | | |

**Tabelle 2 :**

| ¹³C-NMR spektroskopische Daten von Ala⁰-Actagardine | | | | | |
|---|---|---|---|---|---|
| Amino Säure | CO | Cα | Cβ | Cγ | Andere |
| Ala⁰ | 169.51 | 48.35 | 17.35 | | |
| Ala¹ | 169.27 | 50.80 | 34.21 | - | |
| Ser² | 170.48 | 55.04 | 61.23 | | |
| Gly³ | 168.90 | 43.44 | | - | |
| Trp⁴ | | 54.28 | 27.62 | | 110.41, 123.28, |
| | | | | | 127.08, 111.28, |
| | | | | | 120.83,136.00, |
| | | | | | 118.10, 118.22 |
| Val⁵ | 171.20 | 56.73 | 31.50 | 17.76 19. 02 | |
| Ala⁶ | 170.14 | 53.47 | 32.79 | | |
| Abu⁷ | | 57.83 | 43.95 | 19.96 | |
| Leu⁸ | 171.45 | 51.01 | 41.66 | 24.03 | 22.38 |
| | | | | | 22.62 |
| Abu⁹ | 171.59 | 55.77 | 46.38 | 20.10 | |
| Ile¹⁰ | 170. 96 | 60. 00 | 35.71 | 24.75 | 11. 59 |
| | | | β-Me: 14.70 | | |
| Glu¹¹ | | 55.86 | 24.49 | 30.98 | 173.75 |
| Ala¹² | 170.78 | 55.47 | 35.26 | | |
| Gly¹³ | 170.03 | 44.18 | | | |
| Abu¹⁴ | 168.33 | 55.00 | 55.64 | 6.94 | |
| Val¹⁵ | 170.63 | 60.03 | 30.02 | 19.15 | |
| | | | | 18.564 | |
| Ile¹⁶ | 170.66 | 59.64 | 35.69 | 24.55 | 10.62 |
| | | | β-Me: 15.53 | | |
| Ala¹⁷ | 169.79 | 53.13 | 35.70 | | |
| Ala¹⁸ | 171.52 | 48.89 | 15.34 | | |
| Ala¹⁹ | | 47.16 | 51.53 | | |
| unassigned | 169.01 | | | | |
| | 169.83 | | | | |
| | 170.33 | | | | |
| | 171.01 | | | | |

Die Aminosäurenanalyse ergibt zusätzlich zu den Aminosäuren des Actagardines [ 1 Ser, 2 Gly, 1 Trp, 2 Val, 1 Leu, 2 Ile, 1 Glu, 1 Ala, 1 Lanthionin (Ala-S-Ala) und 3 β-Methyllanthionin (Abu-S-Ala)] ein weiteres Ala.

Es wurde weiterhin gefunden, daß die erfindungsgemäße Verbindung starke antibakterielle Wirkungen aufweist, Tabelle 3 faßt die Minimalen Hemmkonzentrationen (MHK) von Ala(0)-Actagardines beispielhaft zusammen.

**Tabelle 3**

| In-Vitro Aktivität des Ala(0)-Actagardines gegen grampositive und anaerobe Bakterien im Reihenverdünnungstest. | |
|---|---|
| KEIM | Ala(0)-Actagardine MHK-Werte (µg/mL) |
| Staph. aureus SG 511 | 6.25 |
| Staph. aureus 285 | 6.25 |
| Staph. aureus 503 | 3.13 |
| Staph. aureus FH 1982 | 12.5 |
| Staph. aureus 701 E | 12.5 |
| Staph. aureus 707 E | 12.5 |
| Staph. aureus 9 Tüb. | 6.25 |
| Staph. aureus 8236 | 6.25 |
| S. epidermidis ZH2c | 6.25 |
| S. epidermidis 6098W | 12.5 |
| S. epidermidis 763 | 6.25 |
| S. epidermidis 5747IIW | 6.25 |
| S. epidermidis 291 | 12.5 |
| S. epidermidis 799 | 6.25 |
| E. faecium Md8B | 6.25 |
| E. faecium VR1 | 50 |
| E. faecium VR2 | 50 |
| S. pyogenes VR3 | 25 |
| S. pyogenes 308A | 6.25 |
| S. pyogenes 77A | 0.195 |
| | |
| Propionib. acnes 6919 | 1.0 |
| Propionib. acnes 6922 | 1.0 |
| Clostrid. tetani 9406 | 8.0 |
| Clostrid. perfringens 194 | 0.5 |

Es ist besonders bemerkenswert, daß die erfindungsgemäße Verbindung nicht nur rund doppelt so gute antibakterielle Wirksamkeiten gegen grampositive Keime aufweist wie das Actagardine, sondern gleichzeitig keinerlei Kreutzresistenz mit herkömmlichen Antibiotika, wie zum Beispiel den β-Lactamen ( Penicilline, Cephalosporine ), Aminoglycosiden ( Streptomycin ), Makroliden ( Erythromycin ), Chinolonen ( Ciprofloxacin ), Sulfonamiden oder Glycopeptiden ( Vancomycin ) und anderen aufweist. Hervorzuheben ist darüber hinaus die starke Hemmwirkung auf Anaerobier, die hartnäckige, ja sogar lebensbedrohende Infektionskrankheiten verursachen können. Zur Therapie solcher Erkrankungen eignet sich das Ala(0)-Actagardine im besonderem Maß.

Die Verträglichkeit des Ala(0)-Actagardines ist in der wirksamen Konzentration und darüber gut. Cytotoxische Wirkungen oder andere Toxizitäten wurden nicht beobachtet.

Die vorliegende Erfindung betrifft demzufolge auch die Anwendung der erfindungsgemäßen Verbindungen als Arzneimittel, sowie die Verwendung der betreffenden Verbindungen zur Herstellung von Arzneimitteln zur Behandlung und/oder Prophylaxe von bakteriellen Infektionen.

Desweiteren betrifft die vorliegende Erfindung Arzneimittel mit einem Gehalt an der erfindungsgemäßen Verbindung.

Das besagte Arzneimittel wird durch Mischung von mindestens einer Verbindung der Formel I mit einem physiologischen Hilfs- und/oder Trägerstoff hergestellt und in eine geeignete Darreichungsform gebracht.

Die erfindungsgemäßen Arzneimittel können enteral (oral), parenteral (intramuskulär oder intravenös), rektal oder lokal (topisch) angewendet werden. Sie können in Form von Lösungen, Pulvem (Tabletten, Kapseln einschließlich Mikrokapseln), Salben (Cremes oder Gel), oder Suppositorien verabreicht werden. Als Hilfsstoffe für derartige Formulierungen kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage. Als zweckmäßige Dosierung werden 0.1 - 1000, vorzugsweise 0.2 - 100 mg/kg Körpergewicht verabreicht. Sie werden zweckmäßig in Dosierungseinheiten verabreicht, die mindestens die wirksame Tagesmenge der erfindungsgemäßen Verbindungen, z. B. 30 - 3000, vorzugsweise 50 - 1000 mg enthalten.

Durch die nachfolgenden Ausführungsbeispiele sowie durch den Inhalt der Patentansprüche soll die vorliegende Erfindung näher erläutert werden.

### Beispiel 1: Herstellung einer Mycelsuspension des Produzenten-Stammes

100 ml Nährlösung (10 g Stärke, 10 g Glycerin, 10 g Glucose, 2,5 g Comsteep, 5 g Pepton und 2 g Hefeextrakt in 1 L Leitungswasser, pH-Wert vor der Sterilisation: 6,0) in einem 500 ml sterilen Erlenmeyerkolben werden mit dem Stamm beimpft und 72 Stunden bei 28°C und 140 UpM auf einer rotierenden Schüttelmaschine inkubiert. Anschließend werden 120 ml Kulturflüssigkeit in einem sterilen 500 ml Erlenmeyerkolben mit dem Nährboden Hafermehlinfus, 2,0 g/L, dem zusätzlich 15 g Agar/L zur Verfestigung zugegeben wurde, gleichmäßig verteilt und dekantiert. Die Kulturen werden 10 bis 14 Tage bei 28° C inkubiert. Das nach dieser Zeit entstandene Mycel eines Kolbens wird ausgestochen, sofort weiterverwendet oder bei -22°C in 50 % Glycerin oder in 10 % Dimethylsulfoxid bei -140° C aufbewahrt.

### Beispiel 2: Herstellung einer Kultur bzw. einer Vorkultur des Produzentenstammes im Erlenmeyerkolben.

Ein steriler 500 ml Erlenmeyerkolben mit 100 ml der im Beispiel 1 beschriebenen Nährlösung wird mit einer auf einem Schrägröhrchen gewachsenen Kultur oder mit einem Agarstück angeimpft und auf einer Schüttelmaschine im Dunkeln bei 140 UpM und 28°C inkubiert. Die maximale Produktion der Verbindungen der Formel I ist nach ca. 72 Stunden erreicht. Zum Animpfen von 10 und 100 L Fermentem genügt eine 72 Stunden alte Submerskultur (Animpfmenge ca. 5 %) aus der gleichen Nährlösung.

### Beispiel 3: Herstellung des Ala(0)-Actagardines. (SEQ ID NO:1)

Ein 10 L Fermenter wird unter folgenden Bedingungen betrieben:

| | |
|---|---|
| Nährmedium | 2 % Sojamehl |
| | 2 % Mannit |
| Inkubationszeit | 24 oder 48 Stunden |
| Inkubationstemperatur | 28°C |
| Rührergeschwindigkeit | 200 UpM |
| Belüftung | 5 L Luft/min. |

Durch wiederholte Zugabe weniger Tropfen ethanolischer Polyollösung kann die Schaumbildung unterdrückt werden. Das Produktionsmaxiumum wird nach 48 Stunden erreicht.

### Beispiel 4: Isolierung des Antibiotikums Ala(0)-Actagardines.

27 L der nach Beispiel 3 gewonnenen Kulturlösung werden abzentrifugiert und das klare Kulturfiltrat auf eine 3 L fassende, mit dem Adsorptionsharz MCI Gel7 CHP20P gefüllte Säule aufgetragen. Säulenmaße: Breite x Höhe: 11,3 cm x 30 cm. Eluiert wird mit einem Lösungsmittel-Gradienten von 5 % Isopropanol in Wasser bis 50 % Isopropanol und der Säulenausfluß in Fraktionen je 2 L aufgefangen. Die Ala(0)-Actagardinhaltigen Fraktionen, die durch HPLC-Analysen überprüft werden, werden gesammelt und im Vakuum konzentriert sowie gefriergetrocknet (4 g).

### Beispiel 5: Hochdruckflüssigkeitschromatographie (HPLC) des Ala(0)-Actagardines

| | |
|---|---|
| Säule | Nucleosil7 100 - 5 C₁₈AB, 250/4. |
| Mobile Phase | 32 % Acetonitril in 10 mM KaliumPhosphat-Puffer pH 7. |
| Flußgeschwindigkeit | 1 mL pro Minute |
| Detektion durch UV-Absorption bei 210 nm. | |

Es wurde für Ala(0)-Actagardine die Retentionszeit von 16 Min. 50 Sekunden, für Actagardine selber 11 Min. und 20 Sekunden gefunden.

### Beispiel 6: Anreicherung des Ala(0)-Actagardines.

3 g des nach Beispiel 4 gewonnenen Produktes werden auf eine 3 Liter fassende mit Fractogel7 TSK HW-40 s gefüllte Säule (Breite x Höhe = 10 cm x 50 cm). aufgetragen. Das Laufmittel, 50 % Methanol in Wasser wird mit einer Flußrate von 50 ml pro Minute über die Säule gepumpt und der Säulenausfluß fraktioniert (65 ml) aufgefangen. In den Fraktionen 24 bis 28 befindet sich hauptsächlich das Antibiotikum Ala(0)-Actagardine, 140mg.

### Beispiel 7: Endreinigung des Ala(0)-Actagardines.

Das angereicherte Antibiotikum Ala(0)-Actagardin (280 mg), gewonnen nach Beispiel 6, wird auf einer Nucleosil7 12C₁₈AB-HPLC-Säule (Breite x Höhe = 3,2 cm x 25 cm) im Gradientenverfahren mit 5 % bis 30 % Acetonitril in 0,05 % Trifluoressigsäure aufgetrennt. Die durch analytische HPLC (siehe Beispiel 5) untersuchten Fraktionen werden entsprechend zusammengefaßt, im Vakuum eingeengt und gefriergetrocknet. Sie ergeben 185 mg Ala(0)-Actagardine in 98 %iger Reinheit.

Durch ESI+-Massenspektrometrie ermitteltes Molekulargewicht des Ala(0)-Actagardines: M+H⁺ = 1962.6.

### Beispiel 8: Gewinnung des Lys(0)-Actagardines. (SEQ ID NO: 2)

In 10 ml wasserfreiem Dimethylformamid (DMF) werden 94.5 mg (0.05 mmol) Actagardine gelöst und 22 mg (0.05 mmol) di-Boc-Lysin-O-N-Hydroxysuccinimid sowie 100 Fl Triethylamin (TEA) hinzu gegeben und bei Raumtemperatur stehen gelassen. Der Verlauf der Umsetzung wird analytisch mittels HPLC (siehe Beispiel 5) verfolgt. Nach 96 Stunden wird die Reaktion durch Abziehen des DMF und des TEA im Hochvakuum unterbrochen und das Umsetzungsprodukt durch präparative HPLC im Gradienten-Verfahren mit 25 bis 50 % Acetonitril in 0.05 %iger Trifluoressigsäure (TFA) gereinigt. Die Säulenmaße betragen Höhe x Breite: 25 mm x 250 mm; Träger: Select B7. Nach Gefriertrocknung der Reaktionsprodukt-enthaltenden Fraktionen werden 33 mg (0.015 mmol) di-Boc-Lys(0)-Actagardine erhalten.
Mit 60 %iger TFA werden die Boc-Schutzgruppen vollständig abgespalten. Hierzu werden 25 mg (0.011 mmol) des geschützten Derivates in 5 ml 60 %iger **TFA** bei Raumtemperatur gelöst. Nach 90 Minuten ist die Abspaltung beendet. Das freie Lysyl-Actagardine wird mit dem gleichen Gradienten, wie oben beschrieben, auf einer präparativen HPLC-Säule (10mm x 250mm, LiChrospher7) gereinigt. Die Gefriertrocknung des gereinigten Materials ergibt 14 mg (0.007 mmol) Lys(0)-Actagardine.
Durch Massenspektrometrie wird das Molekulargewicht des Endproduktes überprüft. Sie beträgt (M+H)⁺:2019, entsprechend der Summenformel C₈₇H₁₃₆O₂₅N₂₂S₄.

### Beispiel 9: Die Herstellung von Ile(0)-Actagardine.

189 mg (0.1 mmol) Actagardine werden wie im Beispiel 8 beschrieben mit 33 mg (0.1 mmol) Boc-Ile-O-N-Hydroxysuccinimid-ester umgesetzt. Es werden 210 mg Boc-Ile(0)-Actagardine erhalten.
Die Abspaltung der Boc-Schutzgruppe und die Endreinigung ergeben 84 mg (0.042 mmol) Ile(0)-Actagardine.
Das massenspektometrisch ermittelte Molekulargewicht beträgt (M+H)⁺ :2004, entsprechend der Summenformel C₈₇H₁₃₅O₂₅N₂₁S₄.

### Beispiel 10: Die Herstellung von N-αAminobutyryl-Actagardine [Abu(0)-Actagardine].

94.5 mg (0.05 mmol) Actagardine werden wie im Beispiel 8 beschrieben mit 16.3 mg (0.05 mmol) N-Boc-αAminobuttersäure-para-nitrophenylester umgesetzt. Es resultieren nach 9 Tagen 54 mg N-Boc-Abu(0)-Actagardine und nach Abspaltung der Schutzgruppe 19 mg (0.01 mmol) N-αAminobutyryl-Actagardine.
Molekular peak (M+H)⁺ : 1976, entsprechend der Summenformel C₈₅H₁₃₁O₂₅N₂₁S₄.

### Beispiel 11: Die Herstellung von Gln(0)-Actagardine. (SEQ ID NO: 5)

94.5 mg (0.05 mmol) Actagardine werden wie im Beispiel 8 beschrieben mit 16.4 mg (0.05 mmol) Boc-Glutamin-paranitrophenyl-ester umgesetzt. Nach entfernen der Schutzgruppe erhält man 38 mg (0.019 mmol) Gln(0)-Actagardine.
Molekular peak (M+H)⁺ : 2019, entsprechend der Summenformel C₈₆H₁₃₂O₂₆N₂₂S₄.

### Beispiel 12: Die Herstellung von Phe(0)-Actagardine. (SEQ ID NO: 6)

94.5 mg (0.05 mmol)-Actagardine werden wie im Beispiel 8 beschrieben mit 15.6 mg (0.05 mmol) Boc-Phe-O-N-Hydroxysuccinimid-ester umgesetzt. Nach entfernen der Schutzgruppe erhält man 26 mg (0.013 mmol) Phe(0)-Actagardine.
Molekular peak (M+H)⁺ : 2038, entsprechend der Summenformel C₉₀H₁₃₃O₂₅N₂₁S₄.

### Beispiel 13: Die Herstellung von Phe-Ala(0)-Actagardine. (SEQ ID NO:7)

94.5 mg (0.05 mmol) Actagardine werden wie im Beispiel 8 beschrieben mit 21.7 mg (0.05 mmol) Boc-Phe-Ala-O-N-Hydroxysuccinimid-ester 3 Stunden lang umgesetzt. Nach entfernen der Schutzgruppe erhält man 37 mg (0.018 mmol) Phe-Ala(0)-Actagardine.
Molekular peak (M+H)⁺ : 2109, entsprechend der Summenformel C₉₃H₁₃₈O₂₆N₂₂S₄.

### Beispiel 14: Die Herstellung von D-Ala(0)-Actagardine. (SEQ ID NO:8)

94.5 mg (0.05 mmol) Actagardine werden wie im Beispiel 8 beschrieben mit 14.5 mg (0.05 mmol) Boc-D-Ala-O-N-Hydroxysuccinimid-ester während 24 Stunden umgesetzt Nach entfernen der Schutzgruppe erhält man 47 mg (0.024 mmol) D-Ala(0)-Actagardine.
Molekular peak (M+H)⁺ : 1961, entsprechend der Summenformel C₈₄H₁₂₉O₂₅N₂₁S₄.

Tabellen 4 bis 6 zeigen die in-Vitro Antikabterielle Aktivität (MHK-Werte[Fg/ml]) des Actagardines (Acta) und der erfindungsgemäßen Verbindungen.

**Tabelle 4:**

| Inkubationszeit: **24 h** | Acta | Ala-Acta | Ile-Acta | Gln-Acta | Phe-Acta | Phe-Ala-Acta | Lys-Acta | Abu-Acta |
|---|---|---|---|---|---|---|---|---|
| *S.aureus* SG511 | 20 | 5 | 1.2 | 20 | 0,6 | 5 | 1.2 | 1.2 |
| *S.aureus* SG511 + 10 % Serum | 40 | 20 | 2.5 | 40 | 5 | 10 | 2.5 | 10 |
| *S.aureus* Exp54146 | >40 | 40 | 2.5 | >40 | 5 | 20 | 10 | 20 |
| *S.pyogenes A561* | >40 | <=0.04 | <=0.04 | 5 | >40 | >40 | <=0.04 | >40 |
| *E.faeccium* M78L | 10 | 5 | 5 | 10 | >40 | 10 | 5 | >40 |
| *E. Coli* | >40 | >40 | >40 | >40 | >40 | >40 | >40 | >40 |

**Tabelle 5**

| Inkubationszeit: **24 h** | Acta | Boc-Ala-Acta | Boc-Ile-Acta | Boc-Gln-Acta | Boc-Phe-Acta | Boc-Phe-Ala-Acta | Boc-Lys-Acta | Boc-Abu-Acta |
|---|---|---|---|---|---|---|---|---|
| *S.aureus* SG511 | 20 | 10 | 20 | 40 | 1.2 | 5 | 2.5 | 5 |
| *S.aureus* SG511 + 10 % Serum | 40 | 40 | 40 | >40 | 10 | 10 | 10 | 10 |
| *S.aureus* Exp54146 | >40 | 40 | >40 | >40 | 5 | 10 | 10 | >40 |
| *S.pyogenes* A561 | >40 | 0.150 | >40 | 0,3 | <=0,04 | >40 | <=0. 04 | >40 |
| *E.faeccium* M78L | >40 | 20 | >40 | 40 | 5 | >40 | 10 | >40 |
| *E. Coli* | >40 | >40 | >40 | >40 | >40 | >40 | >40 | >40 |

**Tabelle 6**

| Inkubationszeit: **18 h** | Ala-Acta | Ile-Acta | Boc-Phe-Acta | Lys-Acta | Boc-Lys-Acta |
|---|---|---|---|---|---|
| *S.aureus* 011HT3 | **20** | **2.5** | 5 | 10 | 10 |
| S.aureus 011HT3+10 % Serum | **20** | **2.5** | 10 | 10 | 20 |
| *S. aureus* 011HT3+50 % Serum | **40** | **5** | 40 | 10 | 40 |
| *S. aureus* 011HT18 | **>40** | **>40** | >40 | >40 | >40 |
| *S. epidermidis* 012G020 | **>40** | **>40** | >40 | >40 | >40 |
| *S.aureus* 011HT1 | **1.1** | **1.2** | 10 | 0.6 | 10 |
| *S.aureus* 011DU5 | **40** | **10** | 40 | 10 | 40 |
| *S.aureus* 011CB20 | **>40** | **40** | >40 | >40 | - |
| *S.aureus* 0121064 | **>40** | **>40** | >40 | >40 | >40 |
| S.epidermidis 012G042 | **>40** | **>40** | >40 | >40 | >40 |
| *Staph.coag.negativ* 012HT5 | **>40** | **>40** | >40 | >40 | >40 |
| *S. pyogenes* 02A1SJ1 | **<=0.04** | **<=0.04** | 0.08 | <=0.04 | 0.08 |
| *S.pyogenes* 02A1UC1 | **<=0.04** | **<=0.04** | <=0.04 | <=0.04 | <=0.04 |
| *S.pyogenes* 02A1FI6 | **<=0.04** | **<=0.04** | <=0.04 | <=0.04 | <=0.04 |
| *Strepto gr*.G 02G0CB2 | **20** | **10** | 2.5 | 5 | 2.5 |
| *S.pneumoniae* 030BI2 | **2.5** | **1.2** | 1.2 | 2.5 | 1.2 |
| *S.milleri* 02milGR12 | **40** | **>40** | 40 | 40 | 5 |
| *S.mitis* 02mitGR16 | **20** | **10** | 20 | 10 | 5 |
| *E.faecalis* 02D2HM9 | **>40** | **40** | 40 | >40 | >40 |
| *E.faecalis* 02D2UC5 | **20** | **40** | 40 | 40 | 40 |
| *E.faecalis* 02D2DU18 | **5** | **5** | 10 | 5 | 10 |
| *E. faecalis* 02D2HT10 | **>40** | **>40** | >40 | >40 | >40 |

## Patentansprüche

1. Verbindung der allgemeinen Formel I wobei R der Rest einer Aminosäure bedeutet, sowie deren physiologisch verträglichen Salze.

2. Verbindung der Formel I gemäß Anspruch 1 worin R der Rest einer natürlichen Aminosäure bedeutet.

3. Verbindung der Formel I gemäß Anspruch 2 worin die Aminosäure Ala, Ile, Lys, Phe oder Val bedeutet.

4. Verbindung der Formel I gemäß Anspruch 1, 2 oder 3 worin die Aminosäure Ala bedeutet.

5. Verbindung der Formel I gemäß einem oder mehreren der Ansprüche 1 bis 4 worin der Aminstickstoff der Aminosäure eine abspaltbare Schutzgruppe trägt.

6. Verbindung der Summenformel: C₈₄H₁₂₉N₂₁O₂₅S₄ (Ala⁰-Actagardine) erhältlich durch Fermentierung von *Actinoplanes liguriae*, DSM 11797 oder *Actinoplanes garbadiensis*, DSM 11796 bzw. einer ihrer Varianten und/oder Mutanten in einem Kulturmedium, bis sich die Verbindung Ala⁰-Actagardine in der Kulturbrühe anhäuft und durch anschließender Isolierung der Verbindung, sowie deren pharmakologisch verträglichen Salze.

7. Chemische Derivate, abgeleitet aus einer Verbindung der Summenformel C₈₄H₁₂₉N₂₁O₂₅S₄ ( Ala⁰-Actagardine), erhältlich durch Fermentierung von *Actinoplanes liguriae*, DSM 11797 oder *Actinoplanes garbadiensis*, DSM 11796 bzw. einer ihrer Varianten und/oder Mutanten in einem Kulturmedium, bis sich die Verbindung Ala(0)-Actagardine in der Kulturbrühe anhäuft und durch anschließende Isolierung der Verbindung und Überführung in chemische Derivate sowie deren pharmakologisch verträglichen Salze.

8. Verfahren zur Herstellung einer Verbindung gemäß einem oder mehreren der Ansprüche 1 bis 5, **dadurch gekennzeichnet, daß** Actagardine mit einer Aminosäure umgesetz wird und gegebenenfalls in ihre pharmakologisch verträglichen Salze überführt wird.

9. Verfahren zur Herstellung einer Verbindung gemäß einem oder mehreren der Ansprüche 1 bis 5, **dadurch gekennzeichnet, daß** der Mikroorganismus *Actinoplanes liguriae*, DSM 11797 oder *Actinoplanes garbadiensis*, DSM 11796 bzw. einer ihrer Varianten oder Mutanten in einem Kulturmedium fermentiert wird, eine Verbindung der Formel I isoliert wird und gegebenenfalls in ihre pharmakologisch verträglichen Salze überführt wird.

10. Verfahren gemäß Anspruch 9, **dadurch gekennzeichnet, daß** man *Actinoplanes liguriae*, DSM 11797 oder *Actinoplanes garbadiensis*, DSM 11796 oder ihre Mutanten und/oder Varianten in eine Kohlenstoff- und Stickstoffquelle sowie die üblichen anorganischen Salze und Spurenelemente enthaltenden Kulturmedien unter aeroben Bedingungen fermentiert.

11. Verfahren gemäß Anspruch 9 oder 10, **dadurch gekennzeichnet, daß** die Fermentierung in einem Nährmedium, das als Kohlenstoffquelle 0,5 bis 5 % Mannit und 0,5 bis 5 % Sojamehl enthält durchgeführt wird.

12. Verfahren gemäß einem oder mehreren der Ansprüche 9 bis 11, **dadurch gekennzeichnet, daß** die Fermentation unter aeroben Bedingungen bei einer Temperatur zwischen 20 und 35°C und bei einem pH zwischen 4 und 10 durchgeführt wird.

13. Verbindung gemäß einem oder meheren der Ansprüche 1 bis 7 zur Anwendung als Arzneimittel.

14. Verwendung einer Verbindung gemäß einem oder mehreren der Ansprüche 1 bis 7 zur Herstellung eines Arzneimittels zur Behandlung und Prophylaxe von bakteriellen Infektionskrankheiten.

15. Arzneimittel mit einem Gehalt an mindestens einer Verbindung gemäß einem oder mehreren der Ansprüche 1 bis 7 und einer oder mehrere physiologisch annehmbare Träger sowie gegebenenfalls geeignete Hilfsstoffe.

16. Verfahren zur Herstellung eines Arzneimittels gemäß Anspruch 15, **dadurch gekennzeichnet, daß** man mindestens eine Verbindung gemäß einem oder mehreren der Ansprüche 1 bis 7 mit einem physiologischen Hilfs- und/oder Trägerstoff in eine geeignete Darreichungsform bringt.

## Claims

1. A compound of the formula I 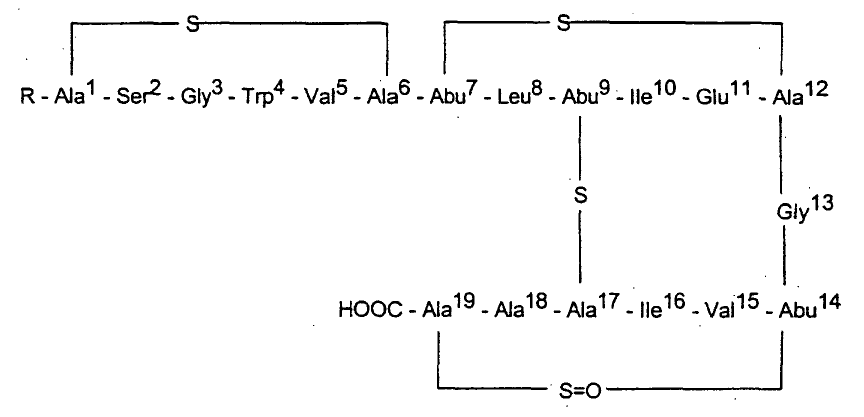 where R is the radical of an amino acid, or its physiologically tolerable salts.

2. A compound of the formula I as claimed in claim 1 in which R is the radical of a natural amino acid.

3. A compound of the formula I as claimed in claim 2 in which the amino acid is Ala, Ile, Lys, Phe or Val.

4. A compound of the formula I as claimed in claim 1, 2 or 3 in which the amino acid is Ala.

5. A compound of the formula I as claimed in one or more of claims 1 to 4 in which the amine nitrogen of the amino acid carries a removable protective group.

6. A compound of the empirical formula: C₈₄H₁₂₉N₂₁O₂₅S₄ (Ala⁰-actagardine) obtainable by fermentation of *Actinoplanes liguriae*, DSM 11797 or *Actinoplanes garbadiensis*, DSM 11796 or one of their variants and/or mutants in a culture medium until the compound Ala⁰-actagardine accumulates in the culture broth and by subsequent isolation of the compound, or its pharmacologically tolerable salts.

7. A chemical derivative derived from a compound of the empirical formula C₈₄H₁₂₉N₂₁O₂₅S₄ ( Ala⁰-actagardine), obtainable by fermentation of *Actinoplanes liguriae*, DSM 11797 or *Actinoplanes garbadiensis*, DSM 11796 or one of their variants and/or mutants in a culture medium until the compound Ala(0)-actagardine accumulates in the culture broth and by subsequent isolation of the compound and conversion into chemical derivatives, or its pharmacologically tolerable salts.

8. A process for the preparation of a compound as claimed in one or more of claims 1 to 5, which comprises reacting actagardine with an amino acid and if appropriate converting it into its pharmacologically tolerable salts.

9. A process for the preparation of a compound as claimed in one or more of claims 1 to 5, which comprises fermenting the microorganism *Actinoplanes liguriae*, DSM 11797 or *Actinoplanes garbadiensis*, DSM 11796 or one of their variants or mutants in a culture medium, isolating a compound of the formula I and if appropriate converting it into its pharmacologically tolerable salts.

10. A process as claimed in claim 9, wherein *Actinoplanes liguriae*, DSM 11797 or *Actinoplanes garbadiensis*, DSM 11796 or their mutants and/or variants are fermented under aerobic conditions in culture media containing a carbon and nitrogen source and also the customary inorganic salts and trace elements.

11. A process as claimed in claim 9 or 10, wherein the fermentation is carried out in a nutrient medium which, as carbon source, contains 0.5 to 5% of mannitol and 0.5 to 5% of soybean flour.

12. A process as claimed in one or more of claims 9 to 11, wherein the fermentation is carried out under aerobic conditions at a temperature between 20 and 35°C and at a pH between 4 and 10.

13. A compound as claimed in one or more of claims 1 to 7 for use as a pharmaceutical.

14. The use of the compound as claimed in one or more of claims 1 to 7 for the production of a pharmaceutical for the treatment and prophylaxis of bacterial infectious diseases.

15. A pharmaceutical containing at least one compound as claimed in one or more of claims 1 to 7 and one or more physiologically acceptable excipients, and, if appropriate, suitable auxiliaries.

16. A process for the production of a pharmaceutical as claimed in claim 15, which comprises bringing at least one compound as claimed in one or more of claims 1 to 7 into a suitable administration form with a physiological auxiliary and/or excipient.

## Revendications

1. Composé de formule générale I 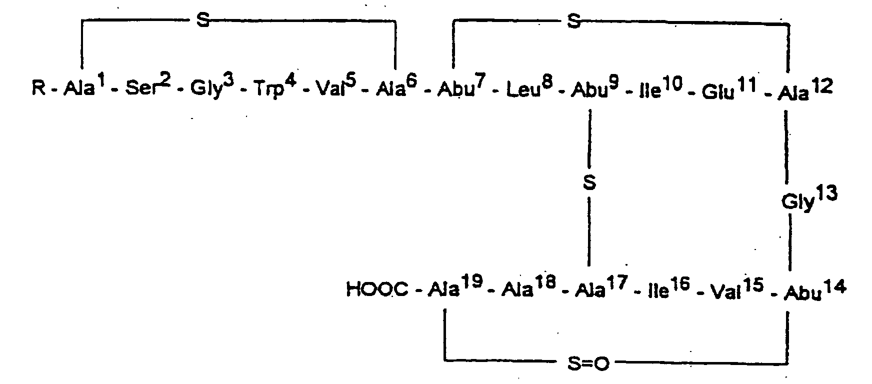 R représente le reste d'un acide aminé, ainsi que ses sels physiologiquement tolérés.

2. Composé de formule I selon la revendication 1, où R représente le reste d'un acide aminé naturel.

3. Composé de formule I selon la revendication 2, où l'acide aminé est Ala, Ile, Lys, Phe ou Val.

4. Composé de formule I selon la revendication 1, 2 ou 3, où l'acide aminé est Ala.

5. Composé de formule I selon une ou plusieurs des revendications 1 à 4, où l'atome d'azote d'amine de l'acide aminé porte un groupe protecteur éliminable.

6. Composé de formule brute C₈₄H₁₂₉N₂₁O₂₅S₄ (Ala⁰-actagardine) que l'on peut obtenir par fermentation d'*Actinoplanes liguriae*, DSM 11797 ou *d'Actinoplanes garbadiensis*, DSM 11796, ou d'une de leurs variantes et/ou mutants dans un milieu de culture, jusqu'à ce que le composé Ala⁰-actagardine s'accumule dans le bouillon de culture et par isolement ultérieur du composé, ainsi que ses sels pharmacologiquement tolérés.

7. Dérivés chimiques, dérivant d'un composé de formule brute C₈₄H₁₂₉N₂₁O₂₅S₄ (Ala⁰-actagardine) que l'on peut obtenir par fermentation d'*Actinoplanes liguriae*, DSM 11797 ou d'*Actinoplanes garbadiensis*, DSM 11796, ou d'une de leurs variantes et/ou mutants dans un milieu de culture, jusqu'à ce que le composé Ala⁰-actagardine s'accumule dans le bouillon de culture et par isolement ultérieur du composé et transformation en dérivés chimiques, ainsi que ses sels pharmacologiquement tolérés.

8. Procédé pour la préparation d'un composé selon une ou plusieurs des revendications 1 à 5, **caractérisé en ce qu'**on fait réagir l'actagardine sur un acide aminé et éventuellement l'on transforme en ses sels pharmacologiquement tolérés.

9. Procédé pour la préparation d'un composé selon une ou plusieurs des revendications 1 à 5, **caractérisé en ce qu'**on fermente le microorganisme *Actinoplanes liguriae*, DSM 11797, ou *Actinoplanes garbadiensis*, DSM 11796, ou une de leurs variantes ou mutants dans un milieu de culture, on isole un composé de formule I et éventuellement on le transforme en ses sels pharmacologiquement tolérés.

10. Procédé selon la revendication 9, **caractérisé en ce qu'**on fermente le microorganisme *Actinoplanes liguriae*, DSM 11797, ou *Actinoplanes garbadiensis*, DSM 11796, ou une de leurs mutants et/ou variantes dans une source de carbone ou d'azote, ainsi que des milieux de culture contenant des sels inorganiques et des oligoéléments usuels sous conditions aérobiques.

11. Procédé selon la revendication 9 ou 10, **caractérisé en ce qu'**on réalise la fermentation dans un milieu nutritif, qui renferme en tant que source de carbone de 0,5 à 5 % de mannitol et de 0,5 à 5 % de farine de soja.

12. Procédé selon l'une des revendications 9 à 11, **caractérisé en ce qu'**on met en oeuvre la fermentation sous conditions aérobiques à une température comprise entre 20 et 35°C et un pH compris entre 4 et 10.

13. Composé selon une ou plusieurs des revendications 1 à 7 pour l'utilisation comme médicament.

14. Composé selon une ou plusieurs des revendications 1 à 7 pour la préparation d'un médicament pour le traitement et la prévention de maladies contagieuses bactériennes.

15. Médicament renfermant au moins un composé selon une ou plusieurs des revendications 1 à 7 et un ou plusieurs véhicules et éventuellement des adjuvants appropriés physiologiquement tolérés.

16. Procédé pour la préparation d'un médicament selon la revendication 15, **caractérisé en ce qu'**on met en forme d'administration appropriée au moins un composé selon une ou plusieurs des revendications 1 à 7 avec un adjuvant et/ou véhicule physiologique.


### SEQUENZPROTOKOLL

(1) ALLGEMEINE INFORMATION:
   (i) ANMELDER:
      (A) NAME: Hoechst Marion Roussel Deutschland GmbH
      (B) STRASSE: -
      (C) ORT: Frankfurt
      (D) BUNDESLAND:-
      (E) LAND: Deutschland
      (F) POSTLEITZAHL: 65926
      (G) TELEPHON: 069-305-40623
      (H) TELEFAX: 069-35-7175
   (ii) ANMELDETITEL: Neues Lantibiotikum verwandt mit Actagardine, Verfahren zur Herstellung und Verwendung derselben
   (iii) ANZAHL DER SEQUENZEN: 8
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
   (v) DATEN DER JETZIGEN ANMELDUNG:
      (A) ANMELDE NUMMER: EP98119105.9
(2) ANGABEN ZU SEQ ID NO: 1:
   (i) SEQUENZKENNZEICHEN;
      (A) LÄNGE: 2 0 Aminosäuren
      (B) ART: Aminosäure
      (C) STRANGFORM: Einzelstrang
      (D) TOPOLOGIE: linear
   (ii) ART DES MOLEKÜLS: Peptid
   (vi) URSPRÜNGLICHE HERKUNFT
      (A) ORGANISMUS: Actinoplanes liguriae und/oder garbadiensis
   (ix) MERKMAL:
      (A) NAME/SCHLÜSSEL: Spezielles Merkmal
      (B) LAGE: 8, 10 und 15
      (D) SONSTIGE ANGABEN: "Xaa = Abu"
   (ix) MERKMAL:
      (A) NAME/SCHLÜSSEL: Thioether-bond
      (B) LAGE: von 2 bis 7
   (ix) MERKMAL:
      (A) NAME/SCHLÜSSEL: Thioether-bond
      (B) LAGE: von 8 bis 13
   ( ix) MERKMAL:
      (A) NAME/SCHLÜSSEL: Thioether-bond
      (B) LAGE: von 10 bis 18
   (ix) MERKMAL:
      (A) NAME/SCHLÜSSEL: Sulfoxide-bond
      (B) LAGE: von 15 bis 20
   (xi) SEQUENZBESCHREIBUNG: SEQ ID NO: 1:
2) ANGABEN ZU SEQ ID NO: 2:
   (i) SEQUENZKENNZEICHEN:
      (A) LÄNGE: 20 Aminosäuren.
      (B) ART: Aminosäure
      (C) STRANGFORM: Einzelstrang
      (D) TOPOLOGIE: linear
   (ii) ART DES MOLEKÜLS: Peptid
   (vi) URSPRÜNGLICHE HERKUNFT
      (A) ORGANISMUS: Actinoplanes liguriae und/oder garbadiensis
   (ix) MERKMAL:
      (A) NAME/SCHLÜSSEL: Spezielles Merkmal
      (B) LAGE: 8, 10 und 15
      (D) SONSTIGE ANGABEN: "Xaa = Abu"
   (ix) MERKMAL:
      (A) NAME/SCHLÜSSEL: Thioether-bond
      (B) LAGE: von 2 bis 7
   (ix) MERKMAL:
      (A) NAME/SCHLÜSSEL: Thioether-bond
      (B) LAGE: von 8 bis 13
   (ix) MERKMAL:
      (A) NAME/SCHLÜSSEL: Thioether-bond
      (B) LAGE: von 10 bis 18
   (ix) MERKMAL:
      (A) NAME/SCHLÜSSEL: Sulfoxide-bond
      (B) LAGE: von 15 bis 20
   (xi) SEQUENZBESCHREIBUNG: SEQ ID NO: 2:
   Lys Ala Ser Gly Trp Val Ala Xaa Leu Xaa Ile Glu Ala Gly Xaa Val
   1               5                   10                  15
   Ile Ala Ala Ala
               20
(2) ANGABEN ZU SEQ ID NO: 3:
   (i) SEQUENZKENNZEICHEN:
      (A) LÄNGE: 20 Aminosäuren
      (B) ART: Aminosäure
      (C) STRANGFORM: Einzelstrang
      (D) TOPOLOGIE; linear
   (ii) ART DES MOLEKÜLS: Peptid
   (vi) URSPRÜNGLICHE HERKUNFT
      (A) ORGANISMUS: Actinoplanes liguriae und/oder garbadiensis
   (ix) MERKMAL:
      (A) NAME/SCHLÜSSEL: Spezielles Merkmal
      (B) LAGE: 8, 10 und 15
      (D) SONSTIGE ANGABEN: "Xaa = Abu"
   (ix) MERKMAL:
      (A) NAME/SCHLÜSSEL: Thioether-bond
      (B) LAGE: von 2 bis 7
   (ix) MERKMAL:
      (A) NAME/SCHLÜSSEL: Thioether-bond
      (B) LAGE: von 8 bis 13
   ( ix) MERKMAL:
      (A) NAME/SCHLÜSSEL: Thioether-bond
      (B) LAGE: von 10 bis 18
   (ix) MERKMAL:
      (A) NAME/SCHLÜSSEL: Sulfoxide-bond
      (B) LAGE: von 15 bis 20
   (xi) SEQUENZBESCHREIBUNG: SEQ ID NO: 3:
   Ile Ala Ser Gly Trp Val Ala Xaa Leu Xaa Ile Glu Ala Gly Xaa Val
   1               5                   10                  15
   Ile Ala Ala Ala
               20
(2) ANGABEN ZU SEQ ID NO: 4:
   (i) SEQUENZKENNZEICHEN:
      (A) LÄNGE: 20 Aminosäuren
      (B) ART: Aminosäure
      (C) STRANGFORM: Einzelstrang
      (D) TOPOLOGIE: linear
   (ii) ART DES MOLEKÜLS: Peptid
   (vi) URSPRÜNGLICHE HERKUNFT
      (A)ORGANISMUS: Actinoplanes liguriae und/oder garbadiensis
   (ix) MERKMAL:
      (A) NAME/SCHLÜSSEL: Spezielles Merkmal
      (B) LAGE: 1, 8, 10 und 15
      (D) SONSTIGE ANGABEN: "Xaa = Abu"
   ( ix) MERKMAL:
      (A) NAME/SCHLÜSSEL: Thioether-bond
      (B) LAGE: von 2 bis 7
   ( ix) MERKMAL:
      (A) NAME/SCHLÜSSEL: Thioether-bond
      (B) LAGE: von 8 bis 13
   (ix) MERKMAL:
      (A) NAME/SCHLÜSSEL: Thioether-bond
      (B) LAGE: von 10 bis 18
   (ix) MERKMAL:
      (A) NAME/SCHLÜSSEL: Sulfoxide-bond
      (B) LAGE: von 15 bis 20
   (xi) SEQUENZBESCHREIBUNG: SEQ ID NO: 4:
   Xaa Ala Ser Gly Trp Val Ala Xaa Leu Xaa Ile Glu Ala Gly Xaa Val
   1               5                   10                  15
   Ile Ala Ala Ala
               20
(2) ANGABEN ZU SEQ ID NO: 5:
   (i) SEQUENZKENNZEICHEN:
      (A) LÄNGE: 20 Aminosäuren
      (B) ART: Aminosäure
      (C) STRANGFORM: Einzelstrang
      (D) TOPOLOGIE: linear
   (ii) ART DES MOLEKÜLS: Peptid
   (vi) URSPRÜNGLICHE HERKUNFT
      (A) ORGANISMUS: Actinoplanes liguriae und/oder garbadiensis
   (ix) MERKMAL:
      (A) NAME/SCHLÜSSEL: Spezielles Merkmal
      (B) LAGE: 8, 10 und 15
      (D) SONSTIGE ANGABEN: "Xaa = Abu"
   (ix) MERKMAL:
      (A) NAME/SCHLÜSSEL: Thioether-bond
      (B) LAGE: von 2 bis 7
   ( ix) MERKMAL:
      (A) NAME/SCHLÜSSEL: Thioether-bond
      (B) LAGE: von 8 bis 13
   (ix) MERKMAL:
      (A) NAME/SCHLÜSSEL: Thioether-bond
      (B) LAGE: von 10 bis 18
   ( ix) MERKMAL:
      (A) NAME/SCHLÜSSEL: Sulfoxide-bond
      (B) LAGE: von 15 bis 20
   (xi) SEQUENZBESCHREIBUNG: SEQ ID NO: 5:
   Gln Ala Ser Gly Trp Val Ala Xaa Leu Xaa Ile Glu Ala Gly Xaa Val
   1               5                   10                  15
   Ile Ala Ala Ala
               20
(2) ANGABEN ZU SEQ ID NO: 6:
   (i) SEQUENZKENNZEICHEN:
      (A) LÄNGE: 20 Aminosäuren
      (B) ART: Aminosäure
      (C) STRANGFORM: Einzelstrang
      (D) TOPOLOGIE: linear
   (ii) ART DES MOLEKÜLS: Peptid
   (vi) URSPRÜNGLICHE HERKUNFT
      (A) ORGANISMUS: Actinoplanes liguriae und/oder garbadiensis
   (ix) MERKMAL:
      (A) NAME/SCHLÜSSEL: Spezielles Merkmal
      (B) LAGE: 8, 10 und 15
      (D) SONSTIGE ANGABEN: "Xaa = Abu"
   (ix) MERKMAL:
      (A) NAME/SCHLÜSSEL: Thioether-bond
      (B) LAGE: von 2 bis 7
   (ix) MERKMAL:
      (A) NAME/SCHLÜSSEL: Thioether-bond
      (B) LAGE: von 8 bis 13
   (ix) MERKMAL:
      (A) NAME/SCHLÜSSEL: Thioether-bond
      (B) LAGE: von 10 bis 18
   (ix) MERKMAL:
      (A) NAME/SCHLÜSSEL: Sulfoxide-bond
      (B) LAGE: von 15 bis 20
   (xi) SEQUENZBESCHREIBUNG: SEQ ID NO: 6:
   Phe Ala Ser Gly Trp Val Ala Xaa Leu Xaa Ile Glu Ala Gly Xaa Val
   1               5                   10                  15
   Ile Ala Ala Ala
               20
(2) ANGABEN ZU SEQ ID NO: 7:
   (i) SEQUENZKENNZEICHEN:
      (A) LÄNGE: 21 Aminosäuren
      (B) ART: Aminosäure
      (C) STRANGFORM: Einzelstrang
      (D) TOPOLOGIE: linear
   (ii) ART DES MOLEKÜLS: Peptid
   (vi) URSPRÜNGLICHE HERKUNFT
      (A)ORGANISMUS: Actinoplanes liguriae und/oder garbadiensis
   (ix) MERKMAL:
      (A) NAME/SCHLÜSSEL: Spezielles Merkmal
      (B) LAGE: 9, 11 und 16
      (D) SONSTIGE ANGABEN: "Xaa = Abu"
   (ix) MERKMAL:
      (A) NAME/SCHLÜSSEL: Thioether-bond
      (B) LAGE: von 3 bis 8
   ( ix) MERKMAL:
      (A) NAME/SCHLÜSSEL: Thioether-bond
      (B) LAGE: von 9 bis 14
   (ix) MERKMAL:
      (A) NAME/SCHLÜSSEL: Thioether-bond
      (B) LAGE: von 11 bis 19
   (ix) MERKMAL:
      (A) NAME/SCHLÜSSEL: Sulfoxide-bond
      (B) LAGE: von 16 bis 21
   (xi) SEQUENZBESCHREIBUNG: SEQ ID NO: 7:
   Phe Ala Ala Ser Gly Trp Val Ala Xaa Leu Xaa Ile Glu Ala Gly Xaa
   1               5                   10                  15
   Val Ile Ala Ala Ala
               20
(2) ANGABEN ZU SEQ ID NO: 8:
   (i) SEQUENZKENNZEICHEN:
      (A) LÄNGE: 20 Aminosäuren
      (B) ART: Aminosäure
      (C) STRANGFORM: Einzelstrang
      (D) TOPOLOGIE: linear
   (ii) ART DES MOLEKÜLS: Peptid
   (vi) URSPRÜNGLICHE HERKUNFT
      (A)ORGANISMUS: Actinoplanes liguriae und/oder garbadiensis
   ( ix) MERKMAL:
      (A) NAME/SCHLÜSSEL: Spezielles Merkmal
      (B) LAGE: 1
      (D) SONSTIGE ANGABEN: "Xaa = D-Alanine"
   (ix) MERKMAL:
      (A) NAME/SCHLÜSSEL: Spezielles Merkmal
      (B) LAGE: 8, 10 und 15
      (D) SONSTIGE ANGABEN: "Xaa = Abu"
   (ix) MERKMAL:
      (A) NAME/SCHLÜSSEL: Thioether-bond
      (B) LAGE: von 2 bis 7
   (ix) MERKMAL:
      (A) NAME/SCHLÜSSEL: Thioether-bond
      (B) LAGE: von 8 bis 13
   ( ix) MERKMAL:
      (A) NAME/SCHLÜSSEL: Thioether-bond
      (B) LAGE: von 10 bis 18
   (ix) MERKMAL:
      (A) NAME/SCHLÜSSEL: Sulfoxide-bond
      (B) LAGE: von 15 bis 20
   (xi) SEQUENZBESCHREIBUNG: SEQ ID NO: 8:
   Xaa Ala Ser Gly Trp Val Ala Xaa Leu Xaa Ile Glu Ala Gly Xaa Val
    1               5                  10                  15
   Ile Ala Ala Ala
               20